(19) **Europäisches Patentamt**
**European Patent Office**
**Office européen des brevets**

(11) **EP 3 646 336 B1**

(12) **EUROPEAN PATENT SPECIFICATION**

(45) Date of publication and mention
of the grant of the patent:
**28.06.2023 Bulletin 2023/26**

(21) Application number: **18737165.3**

(22) Date of filing: **21.06.2018**

(51) International Patent Classification (IPC):
***G16H 40/40*** *(2018.01)*

(52) Cooperative Patent Classification (CPC):
**G16H 40/40**

(86) International application number:
**PCT/EP2018/066519**

(87) International publication number:
**WO 2019/002065 (03.01.2019 Gazette 2019/01)**

(54) **A SYSTEM AND A METHOD FOR RENAL REPLACEMENT THERAPY**

EIN SYSTEM UND EIN VERFAHREN ZUR NIERENERSATZTHERAPIE

SYSTEME ET PROCEDE DE THERAPIE DE REMPLACEMENT RENAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2017 SE 1750838**

(43) Date of publication of application:
**06.05.2020 Bulletin 2020/19**

(73) Proprietor: **Gambro Lundia AB**
**226 43 Lund (SE)**

(72) Inventors:
• **NILSSON, Roger**
**SE-243 34 Höör (SE)**
• **ERICSON, Björn**
**226 52 Lund (SE)**
• **STERNBY, Jan**
**SE-222 36 Lund (SE)**
• **HERTZ, Thomas**
**SE-227 32 Lund (SE)**
• **JANSSON, Olof**
**235 38 Vellinge (SE)**
• **HOBRO, Sture**
**226 47 Lund (SE)**
• **LINDGREN, Henrik**
**247 72 Genarp (SE)**
• **FORS, Jonas**
**215 65 Malmö (SE)**
• **HÅKANSSON, Annmargret**
**S-268 76 Kågeröd (SE)**
• **WALLENBORG, Anders**
**SE-237 33 Bjärred (SE)**

(74) Representative: **Sweden SHS IP Office**
**Gambro Lundia AB**
**P.O. Box 10101**
**220 10 Lund (SE)**

(56) References cited:
**WO-A1-98/10856       US-A1- 2012 273 415**

EP 3 646 336 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical field

The present disclosure relates to the field of renal replacement therapy for extracorporeal treatment of blood, and in particular to a system for renal replacement therapy, and a method for managing membrane fouling of a blood treatment unit in a system for renal replacement therapy.

Background

Renal replacement therapy systems are used for patients that for some reason have to treat their blood outside their bodies. An extracorporeal blood treatment can be used to extract undesirable substances or molecules from the patient's blood, and, if necessary, to add desirable substances or molecules to the blood. The extracorporeal blood treatment is accomplished by passing blood through a blood treatment unit, e.g. a dialyzer or a hemofilter. A membrane separates the treatment unit into a blood compartment and a fluid compartment. Blood withdrawn from the patient is introduced into the blood compartment and flows past the membrane. The membrane is semipermeable to selectively allow matter in the blood to flow across the membrane from the blood compartment into the fluid compartment. The membrane also selectively allows matter in the fluid compartment to flow across the membrane into the blood compartment, depending on the type of treatment.

A number of different types of extracorporeal blood treatments may be performed. In an ultrafiltration (UF) treatment, undesirable matter is removed from the blood by convection across the membrane into the fluid compartment. In a hemofiltration (HF) treatment, the blood flows past the membrane as in a UF treatment and desirable matter is added to the blood, typically by dispensing a fluid into the blood either before and/or after it passes through the treatment unit and before it is returned to the patient. In a hemodialysis (HD) treatment, a secondary fluid containing desirable matter is introduced into the fluid compartment of the treatment unit. Undesirable matter from the blood crosses the membrane into the secondary fluid and desirable matter from the secondary fluid may cross the membrane into the blood. In a hemodiafiltration (HDF) treatment, blood and secondary fluid exchange matter as in HD, and, in addition, matter is added to the blood, typically by dispensing a fluid into the treated blood before its return to the patient as in HF.

Coagulation of the blood, also referred to as clotting, is conventionally reduced by using anti-coagulants such as heparin. However, during treatment material from the blood such as clotted blood, proteins etc. may start to clog the membrane of the treatment unit. This phenomenon may be referred to as membrane fouling. The effects on the membrane are a progressive decline in flux and a change of membrane selectivity. Membrane fouling is mainly caused by concentration polarization and protein adsorption or deposition on the surface or in its pores. Concentration polarization is a result of a concentration gradient due to solute accumulation near the membrane surface. This solute accumulation is also referred to as a secondary membrane. Protein adsorption or deposition on the surface or in its pores is caused by proteins that are absorbed or trapped in the pores of the membrane, and thereby changes the membrane properties. When regions of the membrane start to become less effective, action has to be taken to maintain the same efficiency of the treatment.

Membrane fouling has previously been managed by rinsing the blood circuit and changing filter. However, these procedures are time consuming and the treatment has to be stopped.

From WO2014/095073A1 it is known to control the transmembrane pressure (TMP), haematocrit (HCT) or blood density by regulating at least one of the infusion speeds of the substitution fluids added upstream and/or downstream the treatment unit. Thereby a built-up limiting secondary membrane on the membrane in the blood treatment unit may be kept constant to thereby achieve a constant purifying effect of the blood. For example, in the case of a high TMP or a high HKT value of the blood, the infusion rate of the upstream added substitution fluid can be increased until the desired values of the controlled values are reached. However, dilution of the blood upstream the treatment unit changes the blood composition and its effect on the treatment efficiency is debated.

Summary

In HF and HDF treatments, especially high volume HF and HDF treatments, it is in most cases desired to add as much as possible of the substitution fluid downstream of the treatment unit, because the blood thereby becomes more concentrated in the treatment unit with a positive effect on the treatment efficiency.

However, the membrane fouling limits the water permeability through the membrane, and the decreased permeability decreases the convective volume that can be achieved during treatment.

Thus, there is a need for an improved method for managing fouling of a blood treatment unit, and in particular in high volume post-dilution HF and HDF treatments.

The objective of the disclosure is to alleviate at least some of the drawbacks with the prior art. It is a further objective to provide a system and a method for managing membrane fouling of a blood treatment unit. It is a still further objective to provide a system and a method for managing membrane fouling of a blood treatment unit in high volume post-dilution HF and HDF treatments. It is a still further objective to provide a system and a method for managing membrane fouling such that the efficiency of the treatment is increased. It is another objective to reduce the use of heparin or other anti-coagulants during treatment. Some patients cannot sustain anticoagulation drugs and for these patients so called Heparin Free treatments are used. In such treatments, the membrane will be particularly vulnerable to fouling.

These objectives and others are at least partly achieved by the system and the method according to the independent claims, and by the embodiments according to the dependent claims.

According to a first aspect, the disclosure relates to a system for renal replacement therapy comprising a blood treatment unit arranged to be connected to a blood circuit for extracorporeal circulation of blood comprising an arterial line and a venous line. The blood treatment unit comprises a blood compartment and a fluid compartment separated by a semipermeable membrane. The system further comprises a blood pump arranged to create a blood flow rate $Q_B$ in the arterial line, a pre-dilution line arranged for infusion of substitution fluid upstream the treatment unit, a post-dilution line arranged for infusion of substitution fluid downstream the treatment unit, flow control means arranged to regulate a pre-dilution flow rate $Q_{pre}$ of substitution fluid in the pre-dilution line and a post-dilution flow rate $Q_{post}$ of substitution fluid in the post-dilution line. The system further comprises a control unit configured to control the system according to control instructions. The control instructions comprise to, during a treatment, determine a system parameter value and an indication of membrane fouling of the membrane based on a predetermined criterion for the determined system parameter value, and to activate an automatic anti-fouling measure comprising a temporary increase of the pre-dilution flow rate $Q_{pre}$ and a temporary decrease of the post-dilution flow rate $Q_{post}$ from their present flow rates, respectively, during at least one time period $\Delta t1$ in order to create a flushing of the blood treatment unit, wherein the temporary increase comprises an increase of the pre-dilution flow rate $Q_{pre}$ to more than 100% of a value the post-dilution flow rate $Q_{post}$ had before the anti-fouling measure was activated.

With the described system, the membrane fouling is counteracted, and the membrane fouling is decreased. It is thereby possible to achieve an overall more efficient treatment. As the pre-dilution flow rate is increased during a predetermined short time period $\Delta t1$, the addition of pre-dilution fluid can be kept low and thus support a treatment with high efficiency. The membrane becomes more permeable whereby the effect on the ultrafiltration rate in HF and HDF treatments can be decreased, i.e. it is possible to keep a higher ultrafiltration rate, and thereby an overall higher post-dilution flow rate. In comparison with having a pre-dilution flow rate for a longer period of time where the amount of added pre-dilution fluid becomes so large that it changes the properties of the treatment, the described temporary increase of pre-dilution flow rate and decrease of post-dilution flow rate during the same time period $\Delta t1$ gives the advantage of a treatment with a high efficiency with low or short influence on the treatment properties. Further, less or no heparin may be used which is beneficial for most patients and especially for patients who cannot use heparin.

As a further explanation: When adding fluid upstream the blood treatment unit, the blood becomes thinner and much easier to filtrate. An ultrafiltration rate of the system may then be increased. However, since the blood is diluted, the "system clearance", i.e. the clearance of undiluted blood, will be reduced by the pre-dilution factor. The system according to the disclosure enables an average high ultrafiltration rate, while at the same time delivering an average high system clearance.

According to some embodiments, the anti-fouling measure comprises a temporary stop of the post-dilution flow rate rate $Q_{post}$ during the at least one time period $\Delta t1$.

According to some embodiments, the system further comprises a dialysis fluid circuit comprising a supply line arranged for supply of a dialysis fluid to the blood treatment unit, and a drain line arranged for withdrawal of used fluid from the blood treatment unit, wherein the anti-fouling measure comprises to perform a backwash comprising to infuse a volume of dialysis fluid from the fluid compartment into the blood compartment through the semipermeable membrane.

According to some embodiments, the anti-fouling measure comprises to synchronize timing of the backwash with timing of the flushing such that the backwash has an impact on the blood treatment unit simultaneously and/or earlier than the flushing has an impact on the blood treatment unit.

According to some embodiments, the predetermined criterion comprises a limit value of the system parameter value, and the control instructions comprise to compare the determined system parameter value with the limit value of the system parameter, and to control the anti-fouling measure based on a result of the comparison.

According to some embodiments, the control instructions comprise to determine a length of the at least one time period $\Delta t1$ based on the result of the comparison.

According to some embodiments, the at least one time period $\Delta t1$ has a length of 0.1-6 seconds.

According to some embodiments, the system parameter to be determined is based on one or several of a current $I_B$ consumed by a motor of the blood pump, a system pressure $P_{bi}$, a transmembrane pressure, TMP, across the blood treatment unit; a haemoconcentration of the blood $C_E$, the post-dilution flow rate $Q_{post}$; an ultrafiltration, UF, coefficient, a clearance K of the treatment and/or another efficiency parameter of the treatment. The system parameter may also be determined based on a combination of any of the specified parameters.

According to some embodiments, the control instructions comprise to control an ultrafiltration rate and/or post-dilution flow rate after an anti-fouling measure, such that any temporary change of the ultrafiltration rate and/or the temporary change of the post-dilution flow rate are compensated for during the remaining treatment. Thereby the anti-fouling measure will not have any impact to the amount of fluid withdrawn from the patient.

According to a second aspect, the disclosure relates to a method for use in a renal replacement therapy system comprising a blood treatment unit arranged to be connected to a blood circuit for extracorporeal circulation of blood and an arterial line and a venous line. The blood treatment unit comprises a blood compartment and a fluid compartment separated by a semipermeable membrane. The system further comprises a blood pump arranged to create a blood flow rate $Q_B$ in the arterial line, a pre-dilution line arranged for infusion of substitution fluid upstream the treatment unit, a post-dilution line arranged for infusion of substitution fluid downstream the treatment unit and flow control means arranged to regulate a pre-dilution flow rate of substitution fluid in the pre-dilution line and a post-dilution flow rate of substitution fluid in the post-dilution line, and a control unit. The method comprises during a treatment: determining a system parameter value; determining an indication of membrane fouling of the membrane based on a predetermined criterion for the determined system parameter value; activating an automatic anti-fouling measure comprising temporarily increasing the pre-dilution flow rate $Q_{pre}$ and temporarily decreasing the post-dilution flow rate $Q_{post}$ from their present flow rates, respectively, during at least one time period $\Delta t1$ in order to create a flushing of the blood treatment unit, wherein the temporarily increasing comprises increasing the pre-dilution flow rate $Q_{pre}$ to more than 100% of a value the post-dilution flow rate $Q_{post}$ had before the anti-fouling measure was activated.

The same described advantages as for the system may be achieved by the method.

According to some embodiments, the anti-fouling measure comprises temporarily stopping the post-dilution flow rate $Q_{post}$ during the at least one time period $\Delta t1$.

According to some embodiments, the anti-fouling measure comprises performing a backwash comprising infusing a volume of dialysis fluid from the fluid compartment into the blood compartment through the semipermeable membrane.

According to some embodiments, the method comprises synchronizing timing of the backwash with timing of the flushing such that the backwash has an impact on the blood treatment unit simultaneously and/or earlier than the flushing has an impact on the blood treatment unit.

According to some embodiments, the predetermined criterion comprises a limit value of the system parameter value, and the method comprises

- comparing the determined system parameter value with a limit value of the system parameter; and
- controlling the anti-fouling measure based on a result of the comparison.

According to some embodiments, the method comprises determining a length of the at least one time period $\Delta t1$ based on a result of the comparison.

According to some embodiments, the method comprises controlling timing of the at least one time period $\Delta t1$ based on a result of the comparison.

According to some embodiments, the determined system parameter is based on one or several of a current $I_B$ consumed by a motor of the blood pump, a system pressure $P_{bi}$, a transmembrane pressure, TMP, across the blood treatment unit; a haemoconcentration of the blood $C_E$, the post-dilution flow rate $Q_{post}$, an ultrafiltration, UF, coefficient, a clearance K of the treatment and/or another efficiency parameter of the treatment. The system parameter may also be determined based on a combination of any of the specified parameters.

According to some embodiments, the method comprises controlling an ultrafiltration rate and/or the post-dilution flow rate after an anti-fouling measure, such that any temporary change of the ultrafiltration rate and/or temporary change of the post-dilution flow rate are compensated for during the remaining treatment.

According to a third aspect, the disclosure relates to a computer program at a system, wherein the computer program comprises computer instructions to cause a control unit to perform the method according to any of the steps as disclosed herein.

According to a fourth aspect, the disclosure relates to a computer program product comprising computer instructions stored on a computer readable medium for performing the method according to any of the steps as disclosed herein.

Brief description of the drawings

Fig. 1 illustrates a system for a renal replacement treatment according to some embodiments.

Fig. 2 shows a diagram illustrating flushing of the membrane according to some embodiments.
Fig. 3 shows a diagram illustrating flushing of the membrane according to another embodiment.
Fig. 4 illustrates a method for a renal replacement treatment system according to some embodiments.

Detailed description

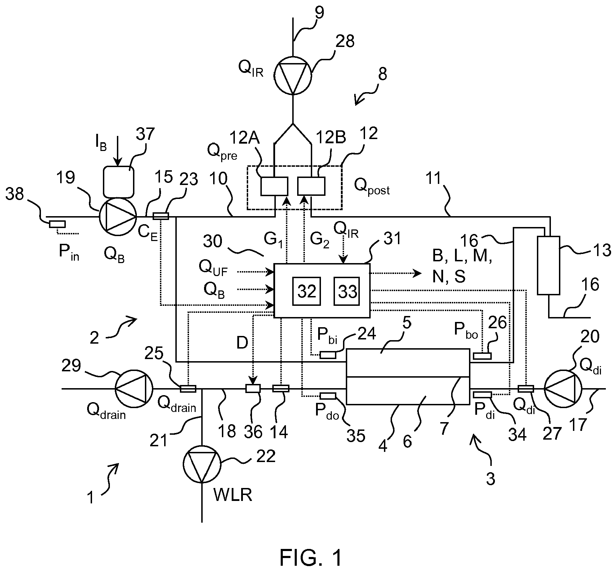

In Fig. 1 a renal replacement therapy system 1 is illustrated. The system 1 comprises an extracorporeal blood circuit 2, a dialysis fluid circuit 3 and a blood treatment unit 4. The blood treatment unit 4 may be a filter unit or a dialyzer. The blood treatment unit 4 comprises a blood compartment 5 and a fluid compartment 6 separated by a semipermeable membrane 7. The semipermeable membrane 7 may be made of a plurality of hollow fibers.

The extracorporeal blood circuit 2 comprises an arterial line 15 connected to an inlet of the blood compartment 5 of the blood treatment unit 4, and a venous line 16 connected to an outlet of the blood compartment 5 of the blood treatment unit 4. The blood treatment unit 4 is thus arranged connected to the blood circuit 2 for extracorporeal circulation of blood. The arterial line 15 is fitted with a blood pump 19 arranged to create a blood flow rate $Q_B$ in the arterial line 15. The blood pump 19 may be a peristaltic blood pump. The venous line 16 is fitted with a drip chamber 13. The arterial line 15 may be fitted with an auxiliary drip chamber (not shown) downstream the blood pump 19. A motor 37 is arranged to rotate the blood pump 19. The motor 37 may be a brushed or brushless DC motor. A motor controller regulates the rotational speed of the blood pump 19. The speed of the blood pump 19, expressed as rotations per minute (RPM), regulates the blood flow rate $Q_B$ in the arterial line 15. The blood pump speed is dependent upon the voltage applied to the motor 37. Further, the blood flow rate $Q_B$ in the arterial line downstream the blood pump 19 is mainly proportional to the blood pump speed. This proportionality is based on the volumetric stroke volume of the blood pump that is virtually constant. However, smaller but sometimes significant deviations of the proportionality between the voltage and the flow exist due to long time use of the pump segment, the pressure $P_{in}$ to the pump, the pump current $I_B$ etc. However, different types of corrections means exist to account for such deviations and can be used if a better prediction of the exact blood flow rate is needed. Thus, the blood flow rate $Q_B$ may be derived from the blood pump speed.

The current $I_B$ consumed by the motor 37 is proportional to the pressure difference over the pump and if the arterial pressure $P_{in}$ is measured (or known to the system) the system pressure $P_{bi}$ can easily be calculated, as will be explained further below. The system pressure $P_{bi}$ may thus be derived from the current $I_B$. The current $I_B$ consumed by the motor 37 may be measured directly with a built in sensor. The voltage may be measured with another built in sensor. The arterial pressure $P_{in}$ is measured with a pressure sensor 38 arranged to the arterial line 15 upstream the blood pump 19.

The dialysis fluid circuit 3 comprises a supply line 17 and a drain line 18. The supply line 17 is connected to an inlet of the fluid compartment 6 of the blood treatment unit 4, and the drain line 18 is connected to an outlet of the fluid compartment 6 of the blood treatment unit 4. The supply line 17 is fitted with a fresh dialysis fluid pump 20 arranged to create a fresh dialysis fluid flow rate $Q_{di}$ and the drain line 18 is fitted with a drain pump 29 arranged to create a used fluid flow rate $Q_{drain}$. In use, the upstream end of the supply line 17 is connected to a source of fresh dialysis fluid (not shown). The downstream end of the drain line 18 is connected to a container (not shown) for accumulation of the used dialysis fluid or to a sink (not shown).

A supplemental line 21 is in some embodiments connected to the drain line 18 between the blood treatment unit 4 and the drain pump 29, and is fitted with an ultrafiltration pump 22 arranged to supply a desired weight loss rate WLR. The ultrafiltration pump 22 is arranged to cause ultrafiltration of plasma water through the membrane 7 of the blood treatment unit 4. Instead of having two pumps 22, 29, a drain line 18 and a supplemental line 21; the flow rate $Q_{drain}$ of the drain pump 29 may be increased to also withdraw the WL amount (thus the WL rate). In operation of the drain pump 29, the flow rate $Q_{drain}$ of the drain pump 29 is then $Q_{di}$ plus the desired WLR. The ultrafiltration pump 22 and the supplemental line 21 are then not necessary.

The drain line 18 may be fitted with a drain flow restriction device 36 for controlling a fluid flow in the drain line 18. The drain flow restriction device 36 may be arranged downstream the blood treatment unit 4, but upstream any supplemental line 21. The drain flow restriction device 36 may be a valve set. The valve set may be an electromechanically actuated valve set.

An infusion line set 8 is connected to the extracorporeal blood circuit 2. The infusion line set 8 comprises a main line 9, which forks into a pre-dilution line 10 and a post-dilution line 11. The pre-dilution line 10 is connected to the arterial line 15, e.g. downstream the blood pump 19, and upstream the blood treatment unit 4. The post-dilution line 11 is connected to the venous line 16, and here the post-dilution line 11 is connected to the venous drip chamber 13 in the venous line 16. The main line 9 is fitted with an infusion pump 28 supplying a flow rate $Q_{IR}$. A flow control means 12 is arranged to regulate a pre-dilution flow rate $Q_{pre}$ of substitution fluid in the pre-dilution line 10 and a post-dilution flow rate of substitution fluid in the post-dilution line 11. The flow control means 12 may comprise a first flow restriction device 12A arranged to the pre-dilution line 10, and a second flow restriction device 12B arranged to the post-dilution line 11. Alternatively, the flow control means 12 may comprise a common flow restriction device (not shown) fitted to the pre-dilution line 10 and the post-dilution line 11. In use, the upstream end of the main line 9 is connected to a source of substitution fluid (not shown). The pre-dilution line 10 is thus arranged for infusion of substitution fluid upstream the treatment unit 4. Further, the post-dilution line 11 is arranged for infusion of substitution fluid downstream the treatment unit 4.

The dialysis fluid and the substitution fluid may be the same kind of fluid, and may come from the same source. The fluids may be electrolyte and/or buffer solutions. The substitution fluid may also be referred to as infusion fluid or replacement fluid.

The renal replacement therapy system 1 further comprises a control circuit 30 comprising a control unit 31. The control unit 31 comprises a processing unit 32 and a memory unit 33. The control unit 31 is configured to control the system 1 according to control instructions. Thus, the control unit 31 is programmed to control the system 1 according to control instructions. The control circuit 30 comprises a sensor 24 arranged to the arterial line 15 directly upstream of the blood treatment unit 4 for supplying a signal $P_{bi}$ correlated to the blood pressure at the inlet of the blood treatment unit 4. The blood pressure in the arterial line 15, i.e. the outlet pressure from the blood pump 19, may alternatively be calculated as a function of the energy consumption of the blood pump 19 e.g. by measuring the power consumed by the motor 37 of the blood pump 19, see e.g. US6,585,675B1. Based e.g. on the current $I_B$ consumed by the motor 37, the torque produced by the motor 37 may be calculated if the speed and the physical parameters of the motor 37 is known. The torque consumed by the motor 37 is a function of the arterial pressure $P_{in}$ (the inlet pressure to the blood pump 19), the system pressure $P_{bi}$ and a torque needed to compress the pumps segment. The system pressure $P_{bi}$ may be expressed as $P_{bi} = (T_{motor} - T_{tube}) \cdot K_{conv} + P_{in}$, where $T_{motor}$ is the total torque output by the motor, $T_{tube}$ is the torque required to compress the pump segment (a constant of the system), and $K_{conv}$ is a conversion constant from torque to pressure for the system. As the $T_{tube}$ and $K_{conv}$, preferably compensated for usage time and temperature, are constant for the system the motor current $I_B$ may be used directly to indicate the blood pump output pressure, thus the system pressure $P_{bi}$, and thus any changes in the system pressure $P_{bi}$. The control circuit 30 further comprises a sensor 26 arranged to the venous line 16 directly downstream from the blood treatment unit 4 for supplying a signal $P_{bo}$ correlated to the blood pressure at the outlet of the blood treatment unit 4. The control circuit 30 further comprises a sensor 34 arranged to the supply line 17 for supplying a signal $P_{di}$ correlated to the dialysis fluid pressure at the inlet of the blood treatment unit 4. The control circuit 30 further comprises a sensor 35 arranged to the drain line 18 for supplying a signal $P_{do}$ correlated to the dialysis fluid pressure at the outlet of the blood treatment unit 4. The control circuit 30 further comprises a sensor 27 arranged to the supply line 17 for supplying a signal $Q_{di}$ correlated to the flow rate of the dialysis fluid at the inlet of the blood treatment unit 4.

The control circuit 30 further comprises a sensor 25 arranged to the drain line 18 for supplying a signal $Q_{drain}$ correlated to the flow rate of the dialysis fluid at the outlet of the blood treatment unit 4 (and downstream any supplemental line 21). The control circuit 30 also comprises a haemoconcentration sensor 23 arranged somewhere along the arterial line 15, either upstream the connection of the pre-dilution line 10 to the arterial line 15, or downstream the connection of the pre-dilution line 10 to the arterial line 15. The haemoconcentration sensor 23 is arranged to generate a haemoconcentration signal $C_E$ correlated to the haemoconcentration of the blood in the arterial line 15 upstream the connection of the pre-dilution line 10 to the arterial line 15, or downstream the connection of the pre-dilution line 10 to the arterial line 15. It should be understood that the described system 1 is merely an example, and the system 1 may not comprise all of the above described sensors. However, the system 1 may alternatively comprise more and/or others than the described sensors.

Although not illustrated in the Fig. 1, the renal replacement therapy system 1 may comprise at least one system for preparation of the dialysis fluid and/or substitution fluid comprising arrangement for e.g. mixing the fluid or fluids. This may be referred to as on-line preparation of the dialysis fluid and/or substitution fluid. In the following disclosure, on-line preparation of the used fluids is assumed. The system may further comprise at least one heater arranged to heat the dialysis fluid and/or the substitution fluid and at least one degassing unit for degassing the dialysis fluid and/or the substitution fluid. The renal replacement therapy system 1 may also comprise various monitoring means comprising sensors, and optionally alarms, for detecting leakage anywhere in the system 1, for detecting abnormal behavior, for monitoring of various parameters such as conductivity, temperature, density, pH and other blood parameters, fluid parameters and/or system parameters.

One or several of the measured parameter values $P_{bi}$, $P_{bo}$, $P_{di}$, $P_{do}$, $Q_{di}$, $Q_{drain}$ and $C_E$ and the set values of various parameters, such as the blood flow rate $Q_B$, the flow rates $Q_{di}$ and $Q_{drain}$ of the drain pump 29 and the fresh dialysis fluid pump 20, the weight loss rate WLR, the ultrafiltration flow rate UFR across the membrane, and the infusion flow rate $Q_{IR}$, any pre-dilution flow rate $Q_{pre}$, any post-dilution flow rate $Q_{post}$ and current $I_B$ are obtained e.g. received by the control unit 31 for controlling the system 1. The control unit 31 is configured to calculate e.g. the TMP, the clearance K, the UF coefficient, the UFR and other parameters based on the received parameters based on control instructions. The TMP may be expressed as a difference in pressure between the blood pressure in the blood compartment 5 and the fluid pressure in the fluid compartment 6. The TMP may thus be determined by calculating the difference between the blood pressure in the blood chamber 5 and the pressure in the fluid chamber 6. The blood pressure in the blood chamber 5 may be determined by $P_{bo}$ or $P_{bi}$, or an average of $P_{bi}$ and $P_{bo}$. The fluid pressure in the fluid chamber 6 may be determined by $P_{do}$ or $P_{di}$, or an average of $P_{do}$ or $P_{di}$. The TMP may be calculated as a difference between any of the $P_{bo}$, $P_{bi}$ or average of ($P_{bi}$ and $P_{bo}$), and any of the $P_{do}$, $P_{di}$ or average of ($P_{di}$ and $P_{do}$). For example, the TMP may be calculated as a difference between the post-filter pressures $P_{bo}$ and $P_{do}$. Alternatively the TMP is calculated as a difference between an average value of the blood pressure in the blood compartment 5 i.e. $P_{bi}$ and $P_{bo}$; and $P_{do}$ downstream the fluid compartment 6 (the post-filter fluid pressure). As a further alternative, the TMP is calculated as a difference between an average value of the blood pressure in the blood compartment 5 i.e. $P_{bi}$ and $P_{bo}$, and an average value of the fluid pressure in the fluid compartment 6, i.e. $P_{di}$ and $P_{do}$. In practice, the control unit 31 emits output signals for controlling the flow control means 12, e.g. the restriction devices 12A and 12B, the infusion pump 28 and in some embodiments the fresh dialysis fluid pump 20, the drain pump 29, the ultrafiltration pump 22 and the blood pump 19, according to control instructions as will be made clear in the following description. For example, the control unit 31 may be configured to generate a control signal M for regulating the fresh dialysis fluid flow rate $Q_{di}$, and a control signal N for regulating the used fluid flow rate $Q_{drain}$. Further, the control unit 31 may be configured to generate a control signal S for regulating the flow rate WLR generated by the ultrafiltration pump 22. The control unit 31 may also be configured to generate a control signal B for regulating the blood flow rate $Q_B$.

The control unit 31 may further be configured to monitor one or several of the above described sensor values over time, and to calculate a change or changes of one or several of the parameters in $\Delta$-values. For example: $\Delta P_{bi}$, $\Delta P_{bo}$, $\Delta P_{di}$, $\Delta P_{do}$, $\Delta Q_{di}$, $\Delta Q_{drain}$, $\Delta C_E$, $\Delta Q_B$, $\Delta Q_{di}$, $\Delta Q_{drain}$, $\Delta WLR$, $\Delta UFR$, $\Delta Q_{IR}$, $\Delta Q_{pre}$, $\Delta Q_{post}$, $\Delta I_B$, $\Delta TMP$, $\Delta K$, $\Delta UF$ coefficient.

The first and second flow restriction devices 12A and 12B may be independently controlled. Thus, the first flow restriction device 12A arranged to the pre-dilution line 10 is arranged to regulate a flow rate $Q_{pre}$ in the pre-dilution line 10. The second flow restriction device 12B arranged to the post-dilution line 11 is arranged to regulate a flow rate $Q_{post}$ in the post-dilution line 11. The flow restriction devices 12A, 12B may be valve sets. The valve sets may be electromechanically actuated valve sets. One or both of the flow restriction devices 12A, 12B may instead be a pump or pumps (not shown) arranged to the pre-dilution line 10 and the post-dilution line 11, respectively.

In use, the flow rate of substitution fluid is regulated by adjusting the delivery, by the infusion pump 28, of the substitution fluid. The control unit 31 is configured to generate a control signal L to regulate the flow rate $Q_{IR}$ of fluid generated by the infusion pump 28. The settings of the flow restriction devices 12A and 12B thereafter determines if the flow should be conducted in the pre-dilution line 10 or in the post-dilution line 11. The control unit 31 may be configured to generate a control signal $G_1$ to regulate the first flow restriction device 12A, and to generate a control signal $G_2$ to regulate the second flow restriction device 12B. According to some embodiments, the pre-dilution flow $Q_{pre}$ and the post-dilution flow $Q_{post}$ are never simultaneous flows. That is, if the first flow restriction device 12A allows a flow $Q_{pre}$, the second flow restriction device 12B stops the flow $Q_{post}$. And vice versa, if the second flow restriction device 12B allows a flow $Q_{post}$, the first flow restriction device 12A stops the flow $Q_{pre}$. Of course, both the first and the second flow restriction device 12A, 12B may stop their flows $Q_{pre}$ and $Q_{post}$, respectively, to achieve no dilution at all. No dilution may also be achieved by controlling the infusion pump 28 to stop the flow $Q_{IR}$. According to some embodiments, the control of the infusion pump 28 and the control of the flow control means 12 are synchronized. "Flow" is here a synonym to "flow rate".

The system 1 may be set in a plurality of different modes to automatically enable different kinds of treatments, for example hemofiltration (HF), hemodiafiltration (HDF) or ultrafiltration (UF). Because of the flow control means 12 and the infusion pump 28, it is possible to switch between post-dilution mode, pre-dilution mode, simultaneous post-dilution and pre-dilution mode, and also no-dilution mode, e.g. UF, without manual intervention, and also during treatment.

In case the system 1 has on-line preparation, the total ultrafiltration (UF) across the membrane 7 may be controlled in two ways by the ultrafiltration pump 22 (or the drain pump 29 if there is no separate ultrafiltration pump 22) and the infusion pump 28; in volume mode and in TMP mode. In both modes the difference between the flow rates $Q_{di}$ + WLR and $Q_{drain}$ is used to control the ultrafiltration pump 22 and the drain pump 29 such that the difference agrees with the desired weight loss rate WLR. WLR in Fig. 1 is set to zero if the ultrafiltration pump 22 and the fluid line 21 are not existent contrary to what is shown in the Fig. 1. In volume mode the infusion pump 28 is kept at a constant flow rate $Q_{IR}$, and the TMP is allowed to vary. In TMP mode the desired TMP is maintained constant by controlling the infusion flow rate $Q_{IR}$ of the infusion pump 28 which will end up at a value such that UFR=WLR+$Q_{IR}$. This relation is thus maintained in both modes. The control unit 31 is configured to control the system 1 to either volume mode or TMP mode.

As soon as the blood treatment starts, the membrane 7 may start to clog caused by the membrane fouling. During the first couple of minutes, proteins in the blood are absorbed or deposited on the surface of the membrane 7 and thus on the surface of the pores of the membrane 7, which leads to a change in membrane behavior. This phenomenon is here referred to as an initial clogging and is normally considered irreversible in nature. Some recent membranes are designed to take account for the initial clogging, such that they reach their nominal filtration properties after formation of the initial clogging.

As the treatment continues the secondary membrane of material is built up on the surface of the membrane 7 caused by the concentration polarization. This secondary membrane can be controlled by means of high shear on the membrane surface, if high shear can be tolerated in operation.

In order to reduce the membrane fouling, the control instructions comprise to, during a treatment, determine a value of a system parameter and an indication of membrane fouling of the membrane 7 in the blood treatment unit 4 based on a predetermined criterion for the determined system parameter value. In response to such an indication, the control instructions comprise to activate an automatic anti-fouling measure comprising a temporary increase of the pre-dilution flow rate $Q_{pre}$ and a temporary decrease of the post-dilution flow rate $Q_{post}$ from their present flow rates, respectively, during at least one time period $\Delta t1$ in order to create a flushing of the blood treatment unit 4. The present value of the pre-dilution flow rate is for example the value of the pre-dilution flow rate just before the anti-fouling measure is activated, a mean value of the pre-dilution flow rate before the anti-fouling measure is activated or a set value of the pre-dilution flow rate. The present value of the post-dilution flow rate is for example the value of the post-dilution flow rate just before the anti-fouling measure is activated, a mean value of the post-dilution flow rate before the anti-fouling measure is activated or a set value of the post-dilution flow rate.

The temporary increase of the pre-dilution flow rate $Q_{pre}$ comprises an increase of the pre-dilution flow rate $Q_{pre}$ to more than 100% of a value the post-dilution flow rate $Q_{post}$ had before the anti-fouling measure was activated. The value is thus the present value of the post-dilution flow rate $Q_{post}$ being a value of the post-dilution flow rate $Q_{post}$ before it is changed because of the anti-fouling measure, e.g. an estimated or measured value of the post-dilution flow rate $Q_{post}$ just before the anti-fouling measure was activated, or a mean value of the pre-dilution flow rate before the anti-fouling measure was activated. The shear force in the blood compartment 5 is then just temporarily raised, but enough to reduce or remove the secondary membrane build-up. The volume of pre-dilution fluid becomes limited and affects the composition of the blood only for a short period of time. The robustness of the treatment can thus be maintained, and as the secondary membrane decreases, the efficiency of the treatment is enhanced. The increase of pre-dilution flow rate $Q_{pre}$ and decrease of the post-dilution flow rate $Q_{post}$ during the limited time period $\Delta t1$ is hereafter referred to as "temporary flushing". The pre-dilution flow rate may be increased from zero flow rate, thus, from no pre-dilution flow rate at all. The above mentioned present pre-dilution flow rate may thus be zero. The automatic anti-fouling measure is performed automatically by the system 1, without any manual intervention. After the anti-fouling measure is finished, the system 1 returns automatically to normal operation with its previous set parameters, or may adapt its parameters to cope for the anti-fouling measure made.

The time period $\Delta t1$ may have a length between about 0.1-6 seconds, and more preferably between 1-3 seconds.

The control instructions may comprise a temporary stop of the post-dilution flow rate $Q_{post}$ during the at least one time period $\Delta t1$. Thus, the pre-dilution flow rate $Q_{pre}$ is more than 100%, and up to 400% of the value the post-dilution flow rate $Q_{post}$ had before the anti-fouling measure was started, during the time period $\Delta t1$, while the post-dilution flow rate $Q_{post}$ is zero, or close to zero, during the time period $\Delta t1$. For example, the pre-dilution flow rate $Q_{pre}$ may be 110%, 130%, 150%, 200%, 250%, 300%, 350% or 400% of the value of the post-dilution flow rate $Q_{post}$ during the time period $\Delta t1$. In some embodiments, the pre-dilution flow rate $Q_{pre}$ may be 100% of the value of the post-dilution flow rate $Q_{post}$. All the mentioned relations of the pre-dilution flow rate $Q_{pre}$ should be understood to be within a margin.

During normal treatment, the pressure in the fluid compartment 6 is generally lower than the pressure in the blood compartment 5, to withdraw excess fluid from the patient. In order to increase the effect of the above described temporary flushing, the negative pressure in the fluid compartment 6 of the blood treatment unit 4 may be reduced, equalized or even reversed during the same at least one time period $\Delta t1$, compared to the pressure in the fluid compartment 6 during normal treatment, such that a temporary decrease of the ultrafiltration rate across the semipermeable membrane 7 is achieved. To reduce the negative pressure in the fluid compartment 6, in relation to the blood pressure in the blood compartment 5, here means to reduce the pressure difference between the blood compartment 5 and the fluid compartment 6 eventually up to the point where the pressure is higher in the fluid compartment 6 compared to the blood compartment 5. These modes, i.e. reduction, equalization or even reversing the negative pressure in the fluid compartment 6 during $\Delta t1$, may be accomplished by regulating the dialysis fluid flow rate in the supply line 17 and/or the used fluid flow rate $Q_{drain}$ in the drain line 18 and/or the WLR in the supplemental line 21. For example, in a HF or HDF-treatment, the control instructions may comprise to temporarily stop the WLR in the supplemental line 21 (assuming the same flow rates $Q_{drain}$ and $Q_{di}$ in HDF). Thereby the pressure in the fluid compartment 6 will be zero or close to zero and the secondary membrane build-up on the membrane 7 in the blood compartment 5 will easier become loose from the membrane 7. Alternatively, the used fluid flow rate $Q_{drain}$ may be reduced or stopped by controlling the drain pump 29 towards or to zero flow rate, in addition to stopping the WLR. The pressure in the fluid compartment 6 will then gradually become less negative (compared to the pressure in the blood compartment 5) as the fresh dialysis fluid pump 20 is still supplying dialysis fluid to the fluid compartment 6. After a short while the pressures in the blood treatment unit 4 will be equalized, thus, the pressure in the fluid compartment 6 will be the same as the pressure in the blood compartment 5. However, as there is still a blood flow in the blood compartment 5, there will be a pressure gradient along the membrane 7. After a further while, the pressure in the fluid compartment 6 becomes greater than the pressure in the blood compartment 5, and dialysis fluid starts to go through the membrane 7 to the blood compartment 5 and thereby pushes the secondary membrane build-up, and any eventual particles trapped in the pores, into the blood compartment 5. The pushed particles and aggregates of particles may then be flushed away by the increased blood flow rate in the blood compartment 5. A filter in the venous drip chamber 13 collects any aggregates that are considered too large to be maintained in the blood before the blood is being return to the patient.

When the pressure in the fluid compartment 6 becomes positively greater than the pressure in the blood compartment 5, the dialysis fluid is pushed into the blood compartment 5. This corresponds to a negative UFR. A controlled infusion of dialysis fluid from the fluid compartment 6, through the membrane 7 and into the blood compartment 5 may be referred to as a backwash of the membrane 7. According to some embodiments, the anti-fouling measure comprises to perform a backwash comprising to infuse a volume of dialysis fluid from the fluid compartment 6 into the blood compartment 5 through the membrane 7. The backwash is thus built-up by temporarily decreasing the ultrafiltration rate. The controlled infusion of dialysis fluid may thus be accomplished by controlling the pressure in the fluid compartment 6 compared to the pressure in the blood compartment 5, such that the pressure in the fluid compartment is positively increased and becomes greater than the pressure in the blood compartment 5. This pressure increase may be accomplished by controlling any of the fresh dialysis fluid pump 20, ultrafiltration pump 22 and/or drain pump 29. For example, as previously explained, the drain pump 29 and the ultrafiltration pump 22 may be controlled to decrease or stop their flow rates $Q_{drain}$ and WLR, respectively, in order to positively increase the pressure in the fluid compartment 6. Alternatively or as a complement, the fresh dialysis fluid pump 20 may be controlled to increase the fresh dialysis fluid flow rate $Q_{di}$ in order to positively increase the pressure in the fluid compartment 6. Alternatively, the pressure increase may be accomplished by controlling one or several valves arranged to regulate the flow rate in the drain line 18, e.g. the flow restriction device 36, and/or the flow rate in the supplemental line 21. The controlled volume of dialysis fluid infused during backwash is according to some examples between 1-40 ml, and more preferably between 2-10 ml. The time period Δt1 during which this volume is infused is between 0.1-6 seconds, and more preferable between 1-3 seconds. If the controlled volume is zero, then there is no backwash, however the negative pressure acting on the secondary membrane from the fluid chamber 6 will decrease and may become zero such that the secondary membrane easier comes loose from the real membrane 7. The backwash may be performed as one continuous separate infusion e.g. during the time period Δt1.

Just before, e.g. a few seconds before, an infusion of dialysis fluid during backwash is performed, the blood pump 19 may be stopped. Thereby the pressure gradient existing in the blood compartment 5 during normal blood flow rate through the blood compartment 5 is reduced. The hereafter conducted backwash will then be essentially equal in all segments of the blood treatment unit 4. As the pressure from pumped blood in the blood compartment 5 to the membrane 7 then is removed, the effect of the backwash may be enhanced. The blood pump 19 may be stopped for a predetermined time length, e.g. the same time length as the time length of the infusion of dialysis fluid during backwash, e.g. the at least one time period Δt1. Instead of stopping the blood pump 19, the speed of the blood pump 19 may be reduced. The pressure gradient in the blood compartment 5 will then not be reduced as much as described above, but the pressure on the membrane 7 from the pumped blood is reduced and the effect of the backwash may be enhanced. After the backwash, the blood pump 19 is resumed to normal operation.

After the automatic anti-fouling measure and outside the time-period Δt1, the flow rates $Q_{di}$, $Q_{drain}$, WLR (if any) $Q_B$, $Q_{IR}$, $Q_{pre}$ and $Q_{post}$ are resumed to normal operation. However, the total ultrafiltration rate UFR may be automatically increased and/or the post-dilution flow rate $Q_{post}$ may be automatically decreased to compensate for the extra volume(s) infused to the blood during the anti-fouling measure, and to compensate for any decreased UFR. For each treatment, a desired UF-volume is determined that should be withdrawn from the patient during the treatment. The actually withdrawn UF-volume is continuously monitored by the system 1, also during the anti-fouling measure. A temporary decrease of the UFR caused by the anti-fouling measure is therefore known and can be compensated for during the remaining treatment. In other words, the control instructions may comprise to control the ultrafiltration rate after an anti-fouling measure, such that any temporary decrease of the ultrafiltration rate and/or increased pre-dilution flow is compensated for during the remaining treatment. For example, if more fluid is introduced into the extracorporeal blood flow than initially calculated, then this amount of more fluid should be withdrawn e.g. by increasing the UFR. Also, the control instructions may comprise to control the post-dilution flow rate after an anti-fouling measure, such that the temporary decrease of the post-dilution flow rate is compensated for during the remaining treatment, e.g. by increasing the post-dilution flow rate. Thereby the anti-fouling measure will not have any impact to the amount of fluid withdrawn from the patient.

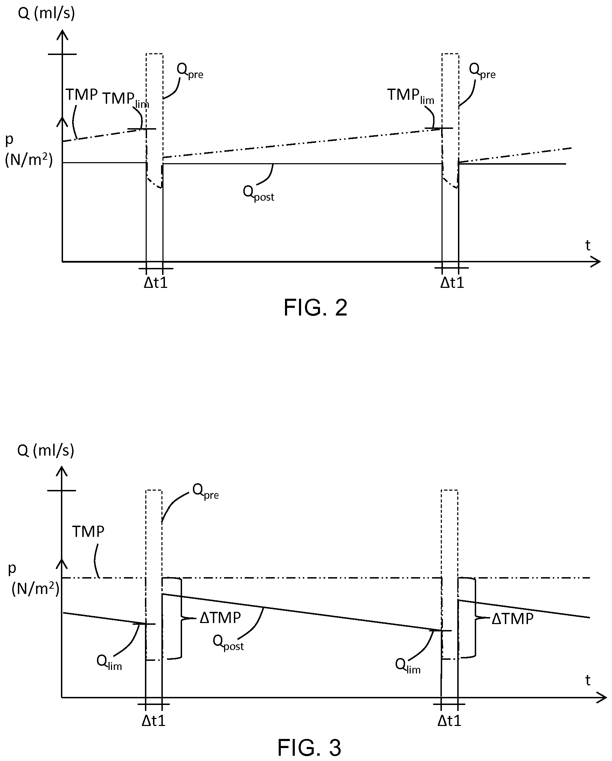

As previously stated, an automatic anti-fouling measure is activated each time the system parameter value is indicating membrane fouling, based on a predetermined criterion for the determined system parameter value. The system parameter to be determined may be based on one or several of the current $I_B$ consumed by a motor 37 of the blood pump 19, the system pressure $P_{bi}$, the ultrafiltration rate UFR, the transmembrane pressure TMP, the haemoconcentration of the blood $C_E$, an UF coefficient, the infusion flow rate $Q_{IR}$, the pre-dilution flow rate $Q_{pre}$, and/or the post-dilution flow rate $Q_{post}$. As has been previously explained, these system parameter values may be sensor values received by the control unit 31, or system parameter values calculated by the control unit 30 by using known equations and received sensor values. According to some embodiments, the predetermined criterion comprises a limit value of the system parameter value. According to some other embodiments, the predetermined criterion comprises a limit value of a change of the system parameter value. The control instructions further comprises to compare the determined system parameter value (or change of system parameter value) with the limit value of the system parameter value (or limit value of the change of the system parameter value), respectively, and to control the anti-fouling measure based on a result of the comparison. Thus, the control instructions may comprise instructions to control activation and/or a length of the at least one time period $\Delta t1$ based on the result of the comparison. Examples of this kind of activation will be explained in the following with reference to Figs. 2-3.

According to some embodiments, the control instructions comprise to synchronize timing of the backwash with timing of the temporary flushing. The timing of the temporary flushing is performed by controlling timing of the temporary increase of the pre-dilution flow rate $Q_{pre}$ and timing of the temporary decrease of the post-dilution flow rate $Q_{post}$. The backwash could for example be synchronized with the temporary flushing, such that the infusion or infusions of dialysis fluid are performed during the same time period or time periods $\Delta t1$ as the temporary flushing. The infusion of the one or several volumes of dialysis fluid is then performed during the same time period or periods $\Delta t1$. Outside the at least one time period $\Delta t1$ the dialysis system 1 is resumed to normal operation with no anti-fouling measure activated. For example, the control instructions comprise to synchronize timing of the backwash with timing of the flushing, such that the backwash has an impact on the blood treatment unit 4 simultaneously as the flushing has an impact on the blood treatment unit 4, during a part of, or during the whole of, the same time period $\Delta t1$. Alternatively, the control instructions comprise to synchronize timing of the backwash with timing of the flushing, such that the backwash has an impact on the blood treatment unit 4 earlier than the flushing has an impact on the blood treatment unit 4. The backwash and the flushing may then have a simultaneous impact on the blood treatment unit 4 during a part of the same time period $\Delta t1$, however, the backwash may first be started and established and thereafter the flushing is performed. The backwash may thus be ongoing when the flushing is started and performed. Alternatively, the backwash is first performed during a first sub-period of $\Delta t1$, and subsequently the flushing is performed during the remaining duration of the time period $\Delta t1$.

The mentioned stopping of the blood pump 19 may be performed during the same time period or time periods $\Delta t1$, such that the blood flow is stopped during the same time period or time periods $\Delta t1$. Alternatively, the blood pump 19 may be stopped during the same time length as $\Delta t1$, but be controlled to be stopped just before, e.g. a few seconds before, the time period $\Delta t1$ of the flushing start. As the pre-dilution flow rate $Q_{pre}$ is infused downstream the blood pump 19, a stop of the blood pump 19 will not influence the pre-dilution flow rate $Q_{pre}$. The fluid in the blood compartment 5 will however be very clean during flushing, that is, comprise almost only the substitution fluid infused during flushing, which may increase the effect of the combined flushing and backwash. Instead of stopping the blood pump 19, the speed of the blood pump 19 may be reduced. The blood flow through the blood compartment 5 will then be reduced and the fluid comprise less concentrated blood than during normal blood flow, which may increase the effect of the combined flushing and backwash. The control unit 31 may be configured to control the speed of the blood pump 19 accordingly, e.g. to reduce the speed of the blood pump 19 or stop the blood pump 19 as described in the foregoing.

According to some embodiments, the infusions of the backwash are only performed during the same time period or time periods $\Delta t1$ as the temporary flushing. The stopping or reduction of the used fluid flow rate $Q_{drain}$ and/or the WLR may also be performed only during the same period of time $\Delta t1$.

In Fig. 2 one treatment example is illustrated in a first diagram of operating the system 1 where the anti-fouling measure, here the temporary flushing, is controlled in dependence of the system parameter TMP in volume mode. The first diagram has flow rate Q on its Y-axis, and time t on its X-axis. A diagram of the TMP of the blood treatment unit 4 is overlaid the first diagram, and has pressure p on its Y-axis and time t on its X-axis. As can be seen from the first diagram, the treatment is in post-dilution mode with a continuous flow rate $Q_{post}$ of substitution fluid added downstream the blood treatment unit 4 and no fluid added upstream the blood treatment unit 4. As the membrane fouling adds on, the TMP increases. When the TMP has reached a predetermined upper $TMP_{lim}$-value, the anti-fouling measure is activated by adding the substitution fluid as a pre-dilution flow rate $Q_{pre}$ while closing off or decreasing the post-dilution flow rate $Q_{post}$ during the time period $\Delta t1$. Here, the pre-dilution flow rate $Q_{pre}$ is 200% of the post-dilution flow rate $Q_{post}$ before the time period $\Delta t1$. Because of the flushing effect, the TMP decreases to a lower value than the $TMP_{lim}$-value. After the time period $\Delta t1$ the post-dilution flow rate $Q_{post}$ resumes its normal rate and the pre-dilution flow rate $Q_{pre}$ is reduced to zero. The TMP gradually increases again, and finally reaches the $TMP_{lim}$-value. The same procedure is then repeated while the treatment is ongoing.

In Fig. 3 another example is illustrated in a second diagram of operating the system 1 where the anti-fouling measure, here the temporary flushing, is controlled in dependence of the amount of substitution fluid added downstream the blood treatment unit 4, thus in dependence of the post-dilution flow rate $Q_{post}$. The second diagram has flow rate Q on its Y-axis, and time t on its X-axis. A diagram of the TMP of the blood treatment unit 4 is overlaid the diagram, and has pressure p on its Y-axis and time t on its X-axis. As can be seen from the second diagram, the treatment is in post-dilution mode with a varying flow rate $Q_{post}$ of substitution fluid added downstream the blood treatment unit 4 and no fluid added upstream the blood treatment unit 4. During this treatment the TMP is held constant and the treatment is thus also in TMP mode. To accomplish a constant TMP, the UFR over the filter 7 is regulated. As the membrane fouling adds on, the UFR at a constant TMP is decreased and in response thereto the post-dilution flow rate $Q_{post}$ is decreased as less substitution fluid has to be added to the extracorporeal blood flow. When the post-dilution flow rate $Q_{post}$ reaches a predetermined lower flow rate $Q_{lim}$, the flushing is activated by adding the substitution fluid as a pre-dilution flow rate $Q_{pre}$ while closing off or decreasing the post-dilution flow rate $Q_{post}$ during the time period $\Delta t1$. Here, the pre-dilution flow rate $Q_{pre}$ is regulated to be about 250% of the rate the post-dilution flow $Q_{post}$ had before the time period $\Delta t1$. Because of the flushing effect, the UFR has to be increased in order to maintain a constant TMP. After the time period $\Delta t1$, the post-dilution flow rate $Q_{post}$ is regulated to a higher value than before the time period $\Delta t1$ to compensate for the increased UFR. Thereafter the post-dilution flow rate $Q_{post}$ gradually decreases again, and finally reaches the predetermined lower flow rate $Q_{lim}$. The same procedure is then repeated. The system parameter may thus be $Q_{post}$ or UFR.

During the time-period of the anti-fouling measure, the treatment mode may be changed from TMP mode to volume mode. After the time-period of the anti-fouling measure, the treatment mode is changed back to TMP mode again. Thereby the desired UFR may still be achieved also during the anti-fouling measure.

Alternatively, the set point for the TMP may be decreased with $\Delta$TMP during the time-period of the anti-fouling measure as illustrated in Fig. 3. After the time-period of the anti-fouling measure, the set point for the TMP is changed back to the previous value. The pressure in the treatment unit 4 will then decrease during the time-period of the anti-fouling measure, whereby the flushing becomes more efficient as the secondary membrane build-up on the membrane 7 will easier come loose from the membrane 7. The set point for the TMP may instead be held constant during the time-period of the anti-fouling measure.

Further, the anti-fouling measure may be activated in response to a current $I_B$ that is higher than a certain current limit $I_{B\_lim}$. A high current $I_B$ corresponds to a correspondingly high system pressure $P_{bi}$. Alternatively, the anti-fouling measure may be activated in response to an ultrafiltration flow rate UFR that is lower than a certain lower ultrafiltration flow rate limit $UFR_{\_lim}$. The anti-fouling measure may also be activated in response to a haemoconcentration value $C_E$ higher than a certain haemoconcentration limit value $C_{E\_lim}$.

It should be understood that instead of comparing absolute values, a change of the system parameter value may be compared with a limit of the change of the system parameter value. Upon the change of the system parameter value is greater than the limit value, the anti-fouling measure is activated.

According to a further embodiment, the anti-fouling measure may be activated in response to a certain efficiency of the treatment, as determined e.g. in EP1729836B1. An efficiency parameter of the treatment may be determined by calculating the UF coefficient as follows:

$$UF\ coefficient = \frac{UFR}{TMP} \qquad (1)$$

The anti-fouling measure may then be activated in response to an UF coefficient lower than a certain UF coefficient limit value $UF_{coeff\_lim}$. Alternatively, an efficiency parameter may be determined by measuring the clearance K of the treatment, i.e. blood water clearance as described in US7435235B2.

In HF clearance K may be calculated from a blood water flow rate $Q_{BW}$, the pre-dilution flow rate $Q_{pre}$, the post-dilution flow rate $Q_{post}$ and a desired weight loss rate WLR of the patient as follows:

$$K = \frac{Q_{BW}}{Q_{BW}+Q_{pre}}(Q_{pre} + Q_{post} + WLR) \qquad (2)$$

The blood water flow rate $Q_{BW}$ is an estimate of the rate of the water in the blood, and is estimated with a factor $f_{BW} \sim 0.85\text{-}0.9$ multiplied with the blood flow rate $Q_B$. Alternatively, the blood flow rate $Q_B$ may be used instead of the blood water flow rate $Q_{BW}$ as an approximation. To calculate the clearance K, the $Q_{BW}$ is then exchanged for $Q_B$ in equation (2). The clearance K may be compared to a limit value of the clearance parameter, and if the clearance K is lower than the limit value, the anti-fouling measure is activated during the time period $\Delta t1$.

The efficiency parameter may instead be the conductivity of the fluid in the drain line 18, or the concentration of at least one substance contained in the fluid in the drain line 18. The system 1 of Fig. 1 is then arranged with a sensor 14 for sensing such a fluid parameter, i.e. conductivity or concentration. The conductivity may be compared to a limit value of the conductivity, and if the conductivity is greater than the limit value, the anti-fouling measure may be activated. Further, concentration of at least one substance may be compared to a limit value of the concentration of the at least one substance, and if the concentration is greater than the limit value, the anti-fouling measure may be activated

The disclosure also relates to a method to be used in a renal replacement therapy system. The method may be performed with the system 1 illustrated in Fig. 1. The system 1 may be arranged for any or all of UF (Ultrafiltration), HF (Hemofiltration) or HDF (Hemodiafiltration) treatment. The system 1 may also be arranged for all the treatments, and the control unit 31 in the system 1 will in dependence on input to the system 1 control the system 1 to perform a certain kind of treatment related to the input. A computer program P is loaded into the memory unit 33 of the control unit 31, and the computer program P comprises computer instructions, that is control instructions, to cause the control unit 31 to perform the method according to any of the method steps as disclosed herein, when the computer program P is run on the processing unit 32. The processing unit 32 may comprise one or several central processing units (CPUs). The computer program P may reside on a computer program product, e.g. a memory unit in a computer or on a server, on a CD-rom, a memory stick or similar.

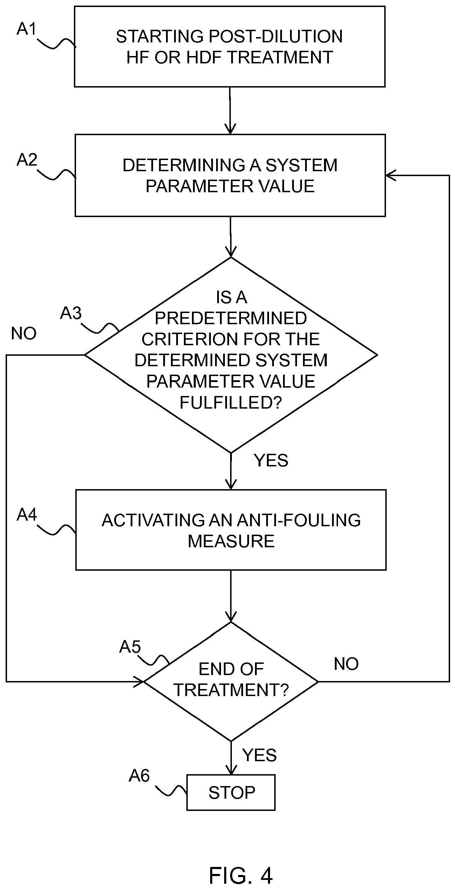

The method will now be described with reference to the flowchart in Fig. 4, and to the system illustration in Fig. 1. Before the present method is activated, a treatment in post-dilution mode has started, such as post-dilution HF or post-dilution HDF, A1. During the treatment, the method comprises A2 determining a system parameter value. The method further comprises determining A3 an indication of membrane fouling of the membrane 7 based on the predetermined criterion for the determined system parameter value. In response to determining such an indication, where the predetermined criterion is fulfilled, the method continues with activating A4 an automatic anti-fouling measure as has been previously described. If the criterion is not fulfilled, and the treatment is not ended A5, the method returns to step A2. The anti-fouling measure comprises A4 temporarily increasing the pre-dilution flow rate $Q_{pre}$ and decreasing the post-dilution flow rate $Q_{post}$ from their present flow rates, respectively, during at least one time period $\Delta t1$ in order to create a flushing of the blood treatment unit 4, wherein the temporarily increasing comprises increasing the pre-dilution flow rate $Q_{pre}$ to more than 100% of a value the post-dilution flow rate $Q_{post}$ had before the anti-fouling measure was activated. The present flow rate of the pre-dilution flow rate $Q_{pre}$ may be zero, thus, the pre-dilution flow rate $Q_{pre}$ may increase from zero flow rate. The anti-fouling measure may comprise temporarily stopping the post-dilution flow rate $Q_{post}$ during the at least one time period $\Delta t1$.

As has been explained, the system parameter value, based on one or several of $I_B$, $P_{bi}$, TMP, $C_E$, $Q_{post}$, UFR, UF coefficient or other efficiency parameter of the treatment, may indicate membrane fouling of the membrane 7, and the temporary flushing may be controlled based on the determined system parameter value $I_B$, $P_{bi}$, TMP, $C_E$, $Q_{post}$, UFR UF coefficient or another efficiency parameter of the treatment. The predetermined criterion may comprise a limit value of the value of the system parameter, or a limit on a change of the system parameter value. The method may comprise comparing the determined system parameter value $I_B$, $P_{bi}$, TMP, $C_E$, $Q_{post}$, UFR or UF coefficient with the limit value of the system parameter, respectively, and controlling the anti-fouling measure based on a result of the comparison. The method may also comprise comparing the determined clearance value K or conductivity of the waste fluid with a limit value of the clearance parameter or conductivity, respectively, and controlling the temporary flushing based on a result of the comparison. Especially timing of the temporary flushing, i.e. activation of the time period $\Delta t1$, may be controlled by monitoring a system parameter or efficiency parameter and activate the temporary flushing when the system parameter value or efficiency parameter goes beyond a certain limit value or range. The at least one time period $\Delta t1$ may have a length of 0.1-6 seconds, preferably 1-3 seconds. The method may comprise determining a length of the time period $\Delta t1$ based on a result of the comparison. For example, if the system parameter value exceeds its limit value with a large amount, the time period $\Delta t1$ may be longer than if the system parameter value exceeds its limit value with a small amount. The method may also comprise determining values for different system parameters, and determining the indication of membrane fouling based on a predetermined criterion each for a plurality of different system parameter values. At least one, a subset of, or all of the system parameter values must then indicate membrane fouling of the membrane 7 in order to activate the automatic anti-fouling measure.

The "temporarily increasing" comprises to temporarily increasing the pre-dilution flow rate $Q_{pre}$ in relation to the amount of post-dilution flow rate $Q_{post}$ before the post-dilution flow rate $Q_{post}$ was decreased. For example, the method may comprise temporarily increasing the pre-dilution flow rate $Q_{pre}$ to more than 100% to 400% of the post-dilution flow rate $Q_{post}$ during the at least one time period $\Delta t1$ of temporary increase of pre-dilution flow rate $Q_{pre}$. Decreasing the post-dilution flow rate $Q_{post}$ may comprise to temporarily stop the post-dilution flow rate $Q_{post}$ during the at least one time period $\Delta t1$. During treatment, the method is continued until the end of treatment A5. If the treatment has stopped, the method is also stopped A6.

The anti-fouling measure may comprise performing a backwash of the membrane 7 of the treatment unit 4 (Fig. 1) as has been previously explained. The backwash may comprise infusing a volume of dialysis fluid to the blood treatment unit 4 in order to enhance the flushing effect on the membrane 7. This means that one or several volumes of dialysis fluid may be infused from the fluid compartment 6 into the blood compartment 5 through the semipermeable membrane 7. The infusions included in the backwash may also be performed in between the time periods $\Delta t1$. The method may comprise synchronizing timing of the backwash with timing of the flushing such that the backwash has an impact on the blood treatment unit 4 simultaneously and/or earlier than the flushing has an impact on the blood treatment unit 4. That is, synchronizing timing such that the infusion or infusions of the volume or volumes of dialysis fluid is/are performed during the at least one time period $\Delta t1$.

Thus, the negative pressure in the fluid compartment 6 of the blood treatment unit 4 may be reduced, equalized or even reversed during the same at least one time period Δt1, compared to the pressure in the fluid compartment 6 during normal treatment. The change in pressure is according to some embodiments performed only during the at least one time period Δt1.

While the invention has been described in connection with what is presently considered to be the most practical and preferred embodiment, it is to be understood that the invention is not to be limited to the disclosed embodiments, but on the contrary, is intended to cover all the modifications comprised within the scope of the appended claims.

**Claims**

1. A system (1) for renal replacement therapy comprising a blood treatment unit (4) arranged to be connected to a blood circuit (2) for extracorporeal circulation of blood comprising an arterial line (15) and a venous line (16), wherein the blood treatment unit (4) comprises a blood compartment (5) and a fluid compartment (6) separated by a semipermeable membrane (7), the system (1) further comprises:

   - a blood pump (19) arranged to create a blood flow rate $Q_B$ in the arterial line (15);
   - a pre-dilution line (10) arranged for infusion of substitution fluid upstream the treatment unit (4);
   - a post-dilution line (11) arranged for infusion of substitution fluid downstream the treatment unit (4);
   - flow control means (12) arranged to regulate a pre-dilution flow rate $Q_{pre}$ of substitution fluid in the pre-dilution line (10) and a post-dilution flow rate $Q_{post}$ of substitution fluid in the post-dilution line (11);
   - a control unit (31) configured to control the system (1) according to control instructions **characterized in that** the control instructions comprise to, during a treatment, determine a system parameter value and an indication of membrane fouling of the membrane (7) based on a predetermined criterion for the determined system parameter value, and activate an automatic anti-fouling measure comprising a temporary increase of the pre-dilution flow rate $Q_{pre}$ and a temporary decrease of the post-dilution flow rate $Q_{post}$ from their present flow rates, respectively, during at least one time period Δt1, in order to create a flushing of the blood treatment unit (4), wherein the temporary increase comprises an increase of the pre-dilution flow rate $Q_{pre}$ to more than 100% of a value the post-dilution flow rate $Q_{post}$ had before the anti-fouling measure was activated.

2. The system (1) according to claim 1, wherein the anti-fouling measure comprises a temporary stop of the post-dilution flow rate $Q_{post}$ during the at least one time period Δt1.

3. The system (1) according to claim 1 or 2, further comprising a dialysis fluid circuit (3) comprising a supply line (17) arranged for supply of a dialysis fluid to the blood treatment unit (4), and a drain line (18) arranged for withdrawal of used fluid from the blood treatment unit (4), and wherein the anti-fouling measure comprises to perform a backwash comprising to infuse a volume of dialysis fluid from the fluid compartment (6) into the blood compartment (5) through the semipermeable membrane (7).

4. The system (1) according to claim 3, wherein the control instructions comprise to control timing of the backwash and timing of the flushing such that the backwash starts earlier than the flushing.

5. The system (1) according to any of the preceding claims, wherein the predetermined criterion comprises a limit value of the system parameter value, and the control instructions comprise to compare the determined system parameter value with the limit value of the system parameter, and to control the anti-fouling measure based on a result of the comparison.

6. The system (1) according to claim 5, wherein the control instructions comprise to determine a length of the at least one time period Δt1 based on the result of the comparison.

7. The system (1) according to any of the preceding claims, wherein the at least one time period Δt1 has a length of 0.1-6 seconds.

8. The system (1) according to any of the preceding claims, wherein the system parameter to be determined is based on one or several of a current $I_B$ consumed by a motor (37) of the blood pump (19), a system pressure $P_{bi}$, a transmembrane pressure, TMP, across the blood treatment unit (4), the post-dilution flow rate $Q_{post}$, an ultrafiltration, UF, coefficient, a haemoconcentration of the blood $C_E$, a clearance K of the treatment and/or another efficiency parameter of the treatment.

9. The system (1) according to any of the preceding claims, wherein the control instructions comprise to control an ultrafiltration rate and/or post-dilution flow rate after an anti-fouling measure, such that any temporary change of the ultrafiltration rate and/or temporary change of the post-dilution flow rate are compensated for during the remaining treatment.

10. A method for use in a renal replacement therapy system (1) comprising a blood treatment unit (4) arranged to be connected to a blood circuit (2) for extracorporeal circulation of blood comprising an arterial line (15) and a venous line (16), wherein the blood treatment unit (4) comprises a blood compartment (5) and a fluid compartment (6) separated by a semipermeable membrane (7), the system (1) further comprises a blood pump (19) arranged to supply a blood flow rate $Q_B$ in the arterial line (15), a pre-dilution line (10) arranged for infusion of substitution fluid upstream the treatment unit (4) and a post-dilution line (11) arranged for infusion of substitution fluid downstream the treatment unit (4), flow control means (12) arranged to regulate a pre-dilution flow rate of substitution fluid in the pre-dilution line (10) and a post-dilution flow rate of substitution fluid in the post-dilution line (11), and a control unit (31), the method comprising during a treatment:

- determining a system parameter value;
- determining an indication of membrane fouling of the membrane (7) based on a predetermined criterion for the determined system parameter value;
- activating an automatic anti-fouling measure comprising temporarily increasing the pre-dilution flow rate $Q_{pre}$ and temporarily decreasing the post-dilution flow rate $Q_{post}$ from their present flow rates, respectively, during at least one time period $\Delta t1$ in order to create a flushing of the blood treatment unit (4), wherein the temporarily increasing comprises increasing the pre-dilution flow rate $Q_{pre}$ to more than 100% of a value the post-dilution flow rate $Q_{post}$ had before the anti-fouling measure was activated.

11. The method according to claim 10, wherein the anti-fouling measure comprises temporarily stopping the post-dilution flow rate $Q_{post}$ during the at least one time period $\Delta t1$.

12. The method according to claim 10 or 11, wherein the anti-fouling measure comprises performing a backwash comprising infusing a volume of dialysis fluid from the fluid compartment (6) into the blood compartment (5) through the semipermeable membrane (7).

13. The method according to claim 12, comprising controlling timing of the backwash and timing of the flushing such that the backwash starts earlier than the flushing.

14. The method according to any of the claims 10 to 13, wherein the determined system parameter is based on one or several of a current $I_B$ consumed by a motor (37) of the blood pump (19), a system pressure $P_{bi}$, a transmembrane pressure, TMP, across the blood treatment unit (4), the post-dilution flow rate $Q_{post}$, an ultrafiltration, UF, coefficient, a haemoconcentration of the blood $C_E$, a clearance K of the treatment and/or another efficiency parameter of the treatment.

15. A computer program at a system (1), wherein the computer program comprises computer instructions that, when executed by a control unit (31), cause the control unit (31) to perform the method according to any of the claims 10 to 14.

**Patentansprüche**

1. System (1) zur Nierenersatztherapie, umfassend eine Blutbehandlungseinheit (4), die vorgesehen ist, um mit einem Blutkreislauf (2) zur extrakorporalen Zirkulation von Blut verbunden zu werden, umfassend eine arterielle Leitung (15) und eine venöse Leitung (16), wobei die Blutbehandlungseinheit (4) eine Blutkammer (5) und eine Fluidkammer (6) umfasst, die durch eine semipermeable Membran (7) getrennt sind, wobei das System (1) des Weiteren umfasst:

- eine Blutpumpe (19), die vorgesehen ist, um eine Blutflussrate $Q_B$ in der arteriellen Leitung (15) zu erzeugen;
- eine Vorverdünnungsleitung (10), die zur Infusion von Substitutionsfluid vorgeordnet zu der Behandlungseinheit (4) vorgesehen ist
- eine Nachverdünnungsleitung (11), die zur Infusion von Substitutionsfluid nachgeordnet zu der Behandlungseinheit (4) vorgesehen ist
- Flusssteuermittel (12), die so vorgesehen sind, dass eine Vorverdünnungsflussrate $Q_{pre}$ des Substitutionsfluids

in der Vorverdünnungsleitung (10) und eine Flussrate nach Verdünnung $Q_{post}$ von Substitutionsfluid in der Nachverdünnungsleitung (11) geregelt werden;

- eine Steuereinheit (31), die ausgestaltet ist, um das System (1) gemäß den Steueranweisungen zu steuern, **dadurch gekennzeichnet, dass** die Steueranweisungen während einer Behandlung Bestimmen eines Systemparameterwerts und einer Angabe von Verunreinigung der Membran (Membran-Fouling) (7) basierend auf einem vorbestimmten Kriterium für den bestimmten Systemparameterwerts und Aktivieren einer automatischen Antifouling-Maßnahme umfassen, umfassend eine zeitweilige Erhöhung der Vorverdünnungsflussrate $Q_{pre}$ und eine zeitweilige Absenkung der Flussrate nach Verdünnung $Q_{post}$ von ihren jeweiligen aktuellen Flussraten während mindestens eines Zeitraums $\Delta t1$, um eine Spülung der Blutbehandlungseinheit (4) zu erzeugen, während die zeitweilige Erhöhung eine Erhöhung der Vorverdünnungsflussrate $Q_{pre}$ auf mehr als 100 % eines Werts umfasst, den die Flussrate nach Verdünnung $Q_{post}$ hatte, bevor die Antifouling-Maßnahme aktiviert wurde.

2. System (1) nach Anspruch 1, wobei die Antifouling-Maßnahme ein zeitweiliges Stoppen der Flussrate nach Verdünnung Qpost während des mindestens einen Zeitraums $\Delta t1$ umfasst.

3. System (1) nach Anspruch 1 oder 2, des Weiteren umfassend einen Dialysefluidkreislauf (3), umfassend eine Zuführleitung (17), die zum Zuführen eines Dialysefluids zu der Blutbehandlungseinheit (4) vorgesehen ist, und eine Drainleitung (18), die zum Abziehen von gebrauchtem Fluid aus der Blutbehandlungseinheit (4) vorgesehen ist, und wobei die Antifouling-Maßnahme Durchführen einer Rückspülung umfasst, umfassend Infundieren eines Volumens an Dialysefluid aus der Fluidkammer (6) in die Blutkammer (5) durch die semipermeable Membran (7) hindurch.

4. System (1) nach Anspruch 3, wobei die Steueranweisungen Steuerung der Zeitwahl der Rückspülung und der Zeitwahl der Spülung umfassen, so dass die Rückspülung früher als die Spülung beginnt.

5. System (1) nach einem der vorhergehenden Ansprüche, wobei das vorbestimmte Kriterium einen Grenzwert des Systemparameterwerts umfasst, und die Steueranweisungen das Vergleichen des bestimmten Systemparameterwerts mit dem Grenzwert des Systemparameters und das Steuern der Antifouling-Maßnahme basierend auf einem Ergebnis des Vergleichs umfassen.

6. System (1) nach Anspruch 5, wobei die Steueranweisungen das Bestimmen einer Länge des mindestens einen Zeitraums $\Delta t1$ basierend auf dem Ergebnis des Vergleichs umfassen.

7. System (1) nach einem der vorhergehenden Ansprüche, wobei der mindestens eine Zeitraum $\Delta t1$ eine Länge von 0,1 bis 6 Sekunden hat.

8. System (1) nach einem der vorhergehenden Ansprüche, wobei der zu bestimmende Systemparameter auf einem oder mehreren von einem Strom IB, der durch einen Motor (37) der Blutpumpe (19) verbraucht wird, einem Systemdruck Pbi, einem Transmembrandruck, TMP, über der Blutbehandlungseinheit (4), der Flussrate nach Verdünnung Qpost, einem Ultrafiltrations-, UF, Koeffizienten, einer Hämatokritkonzentration des Blutes CE, einer Clearance K und/oder einem weiteren Effizienzparameter der Behandlung basiert.

9. System (1) nach einem der vorhergehenden Ansprüche, wobei die Steueranweisungen Steuern einer Ultrafiltrationsrate und/oder einer Flussrate nach Verdünnung nach einer Antifouling-Maßnahme umfassen, so dass jegliche zeitweilige Änderung der Ultrafiltrationsrate und/oder zeitweilige Änderung der Flussrate nach Verdünnung während der verbleibenden Behandlung kompensiert werden.

10. Verfahren zur Verwendung in einem Nierenersatztherapiesystem (1), umfassend eine Blutbehandlungseinheit (4), die vorgesehen ist, um mit einem Blutkreislauf (2) zur extrakorporalen Zirkulation von Blut verbunden zu werden, umfassend eine arterielle Leitung (15) und eine venöse Leitung (16), wobei die Blutbehandlungseinheit (4) eine Blutkammer (5) und eine Fluidkammer (6) umfasst, die durch eine semipermeable Membran (7) getrennt sind, wobei das System (1) des Weiteren eine Blutpumpe (19), die zum Zuführen einer Blutflussrate QB in der arteriellen Leitung (15) vorgesehen ist, eine Vorverdünnungsleitung (10), die zur Infusion von Substitutionsfluid vorgeordnet zu der Behandlungskammer (4) vorgesehen ist, und eine Nachverdünnungsflussleitung (11), die zur Infusion von Substitutionsfluid nachgeordnet zu der Behandlungseinheit (4) vorgesehen ist, Flusssteuermittel (12), die zum Regeln einer Vorverdünnungsflussrate des Substitutionsfluids in der Vorverdünnungsleitung (10) und einer Flussrate nach der Verdünnung von Substitutionsfluid in der Nachverdünnungsleitung (11) vorgesehen ist, und eine Steuereinheit (31) umfasst, wobei das Verfahren während einer Behandlung umfasst:

- Bestimmen eines Systemparameterwerts;
- Bestimmen einer Angabe von Membran-Fouling der Membran (7) basierend auf einem vorbestimmten Kriterium für den bestimmten Systemparameterwert;
- Aktivieren einer automatischen Antifouling-Maßnahme, umfassend zeitweiliges Erhöhen der Vorverdünnungsflussrate $Q_{pre}$ und zeitweiliges Absenken der Flussrate nach Verdünnung $Q_{post}$ von ihren jeweiligen aktuellen Flussraten während mindestens eines Zeitraums $\Delta t1$, um eine Spülung der Blutbehandlungseinheit (4) zu erzeugen, wobei das zeitweilige Erhöhen Erhöhen der Vorverdünnungsflussrate $Q_{pre}$ auf mehr als 100 % eines Wertes umfasst, den die Flussrate nach Verdünnung $Q_{post}$ hatte, bevor die Antifouling-Maßnahme aktiviert wurde.

11. Verfahren nach Anspruch 10, wobei die Antifouling-Maßnahme ein zeitweiliges Stoppen der Flussrate nach Verdünnung Qpost während des mindestens einen Zeitraums $\Delta t1$ umfasst.

12. Verfahren nach Anspruch 10 oder 11, wobei die Antifouling-Maßnahme Durchführen einer Rückspülung umfasst, umfassend Infundieren eines Volumens an Dialysefluid aus der Fluidkammer (6) in die Blutkammer (5) durch die semipermeable Membran (7) hindurch.

13. Verfahren nach Anspruch 12, umfassend Steuerung der Zeitwahl der Rückspülung und der Zeitwahl der Spülung, so dass die Rückspülung früher als die Spülung beginnt.

14. Verfahren nach einem der Ansprüche 10 bis 13, wobei der bestimmte Systemparameter auf einem oder mehreren von einem Strom IB, der durch einen Motor (37) der Blutpumpe (19) verbraucht wird, einem Systemdruck Pbi, einem Transmembrandruck, TMP, über der Blutbehandlungseinheit (4), der Flussrate nach Verdünnung Qpost, einem Ultrafiltrations-, UF, Koeffizienten, einer Hämokonzentration des Blutes CE, einer Clearance K der Behandlung und/oder einem weiteren Effizienzparameter der Behandlung basiert.

15. Computerprogramm an einem System (1), wobei das Computerprogramm Computeranweisungen umfasst, die bei Ausführung durch eine Steuereinheit (31) bewirken, dass die Steuereinheit (31) das Verfahren gemäß einem der Ansprüche 10 bis 14 durchführt.

**Revendications**

1. Système (1) de thérapie de remplacement rénal comprenant une unité de traitement du sang (4) agencée pour être raccordée à un circuit sanguin (2) pour la circulation extracorporelle du sang comprenant une ligne artérielle (15) et une ligne veineuse (16), l'unité de traitement du sang (4) comprenant un compartiment à sang (5) et un compartiment à fluide (6) séparés par une membrane semi-perméable (7), le système (1) comprenant en outre :

- une pompe à sang (19) agencée pour créer un débit sanguin $Q_B$ dans la ligne artérielle (15) ;
- une ligne de pré-dilution (10) agencée pour la perfusion du liquide de substitution en amont de l'unité de traitement (4) ;
- une ligne de post-dilution (11) agencée pour la perfusion du liquide de substitution en aval de l'unité de traitement (4) ;
- des moyens de commande d'écoulement (12) agencés pour réguler un débit de pré-dilution $Q_{pre}$ du fluide de substitution dans la ligne de pré-dilution (10) et un débit de post-dilution $Q_{post}$ du fluide de substitution dans la ligne de post-dilution (11) ;
- une unité de commande (31) configurée pour commander le système (1) selon des instructions de commande **caractérisées en ce que** les instructions de commande comprennent, au cours d'un traitement, la détermination d'une valeur de paramètre de système et d'une indication d'encrassement de membrane de la membrane (7) sur la base d'un critère prédéterminé pour la valeur de paramètre de système déterminée, et d'activer une mesure anti-encrassement automatique comprenant une augmentation temporaire du débit de pré-dilution $Q_{pre}$ et une diminution temporaire du débit de post-dilution $Q_{post}$ par rapport à leurs débits actuels, respectivement, pendant au moins une période $\Delta t1$, afin de créer un rinçage de l'unité de traitement du sang (4), l'augmentation temporaire comprenant une augmentation du débit de pré-dilution $Q_{pre}$ à plus de 100 % d'une valeur que le débit de post-dilution $Q_{post}$ avait avant que la mesure anti-encrassement ne soit activée.

2. Système (1) selon la revendication 1, la mesure anti-encrassement comprenant un arrêt temporaire du débit de post-dilution $Q_{post}$ pendant au moins une période $\Delta t1$.

EP 3 646 336 B1

3. Système (1) selon la revendication 1 ou 2, comprenant en outre un circuit de fluide de dialyse (3) comprenant une ligne d'alimentation (17) agencée pour fournir un fluide de dialyse à l'unité de traitement du sang (4), et une ligne de drainage (18) agencée pour retirer le fluide usagé de l'unité de traitement du sang (4), et la mesure anti-encrassement comprenant la réalisation d'un lavage à contre-courant comprenant la perfusion d'un volume de fluide de dialyse depuis le compartiment à fluide (6) dans le compartiment à sang (5) à travers la membrane semi-perméable (7).

4. Système (1) selon la revendication 3, les instructions de commande comprenant la commande de la synchronisation du lavage à contre-courant et de la synchronisation du rinçage de sorte que le lavage à contre-courant commence plus tôt que le rinçage.

5. Système (1) selon l'une quelconque des revendications précédentes, le critère prédéterminé comprenant une valeur limite de la valeur du paramètre de système, et les instructions de commande comprenant la comparaison de la valeur de paramètre de système déterminée avec la valeur limite du paramètre de système, et la commande de la mesure anti-encrassement sur la base d'un résultat de la comparaison.

6. Système (1) selon la revendication 5, les instructions de commande comprenant la détermination d'une durée de l'au moins une période $\Delta t1$ sur la base du résultat de la comparaison.

7. Système (1) selon l'une quelconque des revendications précédentes, l'au moins une période de temps $\Delta t1$ ayant une longueur comprise entre 0,1 et 6 secondes.

8. Système (1) selon l'une quelconque des revendications précédentes, le paramètre de système à déterminer étant basé sur un ou plusieurs parmi un courant $I_B$ consommé par un moteur (37) de la pompe à sang (19), une pression système $P_{bi}$, une pression transmembranaire, TMP, à travers l'unité de traitement du sang (4), le débit de post-dilution $Q_{post}$, un coefficient d'ultrafiltration, UF, une hémoconcentration du sang $C_E$, une clairance K du traitement et/ou un autre paramètre d'efficacité du traitement.

9. Système (1) selon l'une quelconque des revendications précédentes, les instructions de commande comprenant la commande d'un taux d'ultrafiltration et/ou d'un débit de post-dilution après une mesure anti-encrassement, de sorte que toute modification temporaire du taux d'ultrafiltration et/ou toute modification temporaire du débit de post-dilution soient compensées pendant le reste du traitement.

10. Procédé destiné à être utilisé dans un système de thérapie de remplacement rénal (1) comprenant une unité de traitement du sang (4) agencée pour être raccordée à un circuit sanguin (2) pour la circulation extracorporelle du sang comprenant une ligne artérielle (15) et une ligne veineuse (16), l'unité de traitement du sang (4) comprenant un compartiment à sang (5) et un compartiment à fluide (6) séparés par une membrane semi-perméable (7), le système (1) comprenant en outre une pompe à sang (19) agencée pour fournir un débit sanguin $Q_B$ dans la ligne artérielle (15), une ligne de pré-dilution (10) agencée pour la perfusion du fluide de substitution en amont de l'unité de traitement (4) et une ligne de post-dilution (11) agencée pour la perfusion du fluide de substitution en aval de l'unité de traitement (4), des moyens de commande d'écoulement (12) agencés pour réguler un débit de pré-dilution du fluide de substitution dans la ligne de pré-dilution (10) et un débit de post-dilution du fluide de substitution dans la ligne de post-dilution (11), et une unité de commande (31), le procédé comprenant lors d'un traitement :

- la détermination d'une valeur de paramètre de système ;
- la détermination d'une indication d'encrassement de membrane de la membrane (7) sur la base d'un critère prédéterminé pour la valeur de paramètre de système déterminée ;
- l'activation d'une mesure anti-encrassement automatique comprenant l'augmentation temporaire du débit de pré-dilution $Q_{pre}$ et la diminution temporaire du débit de post-dilution $Q_{post}$ par rapport à leur débit actuel, respectivement, pendant au moins une période $\Delta t1$ afin de créer un rinçage de l'unité de traitement du sang (4), l'augmentation temporaire comprenant l'augmentation du débit de pré-dilution $Q_{pre}$ à plus de 100 % d'une valeur que le débit de post-dilution $Q_{post}$ avait avant que la mesure anti-encrassement ne soit activée.

11. Procédé selon la revendication 10, la mesure anti-encrassement comprenant l'arrêt temporaire du débit de post-dilution $Q_{post}$ pendant l'au moins une période de temps $\Delta t1$.

12. Procédé selon la revendication 10 ou 11, la mesure anti-encrassement comprenant la réalisation d'un lavage à contre-courant comprenant la perfusion d'un volume de fluide de dialyse depuis le compartiment à fluide (6) dans le compartiment à sang (5) à travers la membrane semi-perméable (7).

13. Procédé selon la revendication 12, comprenant la commande de la synchronisation du lavage à contre-courant et de la synchronisation du rinçage de sorte que le lavage à contre-courant commence plus tôt que le rinçage.

14. Procédé selon l'une quelconque des revendications 10 à 13, le paramètre de système déterminé étant basé sur un ou plusieurs parmi un courant $I_B$ consommé par un moteur (37) de la pompe à sang (19), une pression système $P_{bi}$, une pression transmembranaire, TMP, à travers l'unité de traitement du sang (4), le débit de post-dilution $Q_{post}$, un coefficient d'ultrafiltration, UF, une hémoconcentration du sang $C_E$, une clairance K du traitement et/ou un autre paramètre d'efficacité du traitement.

15. Programme d'ordinateur sur un système (1), le programme d'ordinateur comprenant des instructions informatiques qui, lorsqu'elles sont exécutées par une unité de commande (31), amènent l'unité de commande (31) à réaliser le procédé selon l'une quelconque des revendications 10 à 14.

FIG. 1

FIG. 2

FIG. 3

A1 — STARTING POST-DILUTION HF OR HDF TREATMENT

A2 — DETERMINING A SYSTEM PARAMETER VALUE

A3 — IS A PREDETERMINED CRITERION FOR THE DETERMINED SYSTEM PARAMETER VALUE FULFILLED?

NO

YES

A4 — ACTIVATING AN ANTI-FOULING MEASURE

A5 — END OF TREATMENT?

NO

YES

A6 — STOP

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014095073 A1
- US 6585675 B1
- EP 1729836 B1
- US 7435235 B2